Europäisches Patentamt

**European Patent Office**

Office européen des brevets

(11) Veröffentlichungsnummer: **0 128 567**
**A2**

## (12) EUROPÄISCHE PATENTANMELDUNG

(21) Anmeldenummer: **84106606.1**

(22) Anmeldetag: **08.06.84**

(51) Int. Cl.³: **C 12 N 15/00, C 12 N 13/00**

(30) Priorität: **11.06.83 DE 3321226**

(71) Anmelder: **Kernforschungsanlage Jülich Gesellschaft mit beschränkter Haftung, Postfach 1913, D-5170 Jülich (DE)**

(43) Veröffentlichungstag der Anmeldung: **19.12.84 Patentblatt 84/51**

(72) Erfinder: **Zimmermann, Ulrich, Prof, Im Geyberg 21, D-5165 Hürggenwald-Gey (DE)**
Erfinder: **Arnold, William Michael, Adalbertstrasse 116-118, D-5100 Aachen (DE)**
Erfinder: **Büchner, Karl-Heinz, Schützenstrasse 19, D-5170 Jülich (DE)**
Erfinder: **Matschke, Christian, Wirthstrasse 63, D-5110 Alsdorf (DE)**

(84) Benannte Vertragsstaaten: **CH FR GB IT LI**

(54) Verfahren zur Fusion von Zellen.

㊵ Die Erfindung bezieht sich auf ein Verfahren zur Fusion von Zellen, wobei die Zellen zur Erzeugung von Störungen in der Membranstruktur, die zur Bildung von Membranbrükken zwischen einander benachbarten Zellen und zur Fusion der Zellen führen, mit chemischen Mitteln oder inaktivierten Viren behandelt werden. Der Erfindung liegt die Aufgabe zugrunde, bei der Durchführung des Verfahrens eine kontrollierte Fusion zweier oder einer vorbestimmten Anzahl von Zellen zu ermöglichen. Dies wird dadurch erzielt, dass die Zellen zusätzlich zur Behandlung mit den chemischen Mitteln oder den Viren einem äusseren inhomogenen elektrischen Feld derart ausgesetzt werden, dass sich die Zellen in vorbestimmter Ausrichtung aneinanderreihen. Je nach Grösse der Zellen beträgt die Frequenz des anzuwendenden elektrischen Feldes 5 kHz bis 2 MHz und die Stärke des Feldes 10 V/cm bis 2000 V/cm.

ACTORUM AG

0128567

Kernforschungsanlage Jülich
Gesellschaft mit beschränkter Haftung

Verfahren zur Fusion von Zellen

Die Erfindung bezieht sich auf ein Verfahren zur Fusion von Zellen, bei dem die Zellen zur Erzeugung von Störungen in der Membranstruktur, die zur Bildung von Membranbrücken zwischen einander benachbarten Zellen und zur Fusion der Zellen führen, mit chemischen Mitteln, wie Polyethylenglycol, oder mit inaktivierten Viren, wie Sendai-Viren, behandelt werden.

Zwei Zellen in einer Suspension sollten, wenn sie sich berühren und ein enger Kontakt zwischen den Membranen beider Zellen entsteht, miteinander verschmelzen, da die Bausteine in der Membran beweglich sind. Eine derartige spontane Verschmelzung (Fusion) von Zellen wird unter natürlichen Bedingungen jedoch nicht oder nur äußerst selten beobachtet. Eine bekannte Ausnahme stellt die Befruchtung einer Eizelle durch eine Samenzelle bei der sexuellen Fortpflanzung dar. Die spontane Fusion wird durch die negative Ladung der Phospholipide und anderer Membrankomponenten behindert. Sie führt zur Abstoßung der Zellen, wenn sie sich auf einen geringen Abstand genähert haben. Zellverschmelzung erfordert aber, daß die beiden Membranen sich bis auf einen Abstand von weniger

a

- 2 -

- 2 -

als $10^{-9}$ m nähern können.

Die mit technischen Mitteln durchgeführte Fusion von Zellen ist in einem weiten Anwendungsbereich von Interesse. Sie kann beispielsweise eingesetzt werden für die Bildung von Hybridzellen durch Verschmelzung von zwei Zellen unterschiedlicher Herkunft, die evolutionsmäßig nicht zu weit voneinander entfernt stehen sollen. Dabei können Zellhybride aus Pflanzenzellen, aus denen wieder ganze Pflanzen gezüchtet werden können oder Zellhybride aus tierischen Zellen, über die monoklonale Antikörper, z.B. gegen Tumore und Leukämie, gewonnen werden können, gebildet werden. Als Beispiel sei genannt die Verschmelzung einer Lymphozytenzelle mit einer Myelomzelle, die besonders aus medizinischer und pharmazeutischer Sicht von großem Interesse ist. Bestimmte Lymphozyten bilden gegen Fremdstoffe im Organismus Antikörper, z.B. gegen ein Fremdeiweiß, das in die Blutbahn injiziert worden ist. Isoliert man die Lymphozyten und fusioniert sie mit einer Tumorzelle, wie der Myelomzelle, so besteht die Chance, daß sich eine sogenannte Hybridomzelle bildet, die die Eigenschaft beider Elternzellen besitzt. Diese Zelle produziert Antikörper, und zwar spezifisch nur gegen den betreffenden Fremdstoff (sogenannte monoklonale Antikörper). Sie ist unsterblich und läßt sich im Gegensatz zu einer normalen ausdifferenzierten Zelle, wie dem Lymphozyten, permanent in Nährmedien vermehren.

- 3 -

Es ist bekannt, die elektrischen Abstoßungskräfte, die bei geringem Abstand zwischen den
Membranen zweier Zellen auftreten, durch Einsatz
bestimmter Chemikalien, wie Polyethylenglykol
(PEG) oder inaktivierte Viren, auszuschalten.
Sowohl Viren als auch PEG vernetzen die beiden
Zellmembranen, so daß ein enger Membrankontakt
entsteht. Gleichzeitig werden durch die Viren
und das PEG Störungen in der Membranstruktur
erzeugt, die durch Einstellen unphysiologischer
Bedingungen, wie z.B. Zugabe hoher Konzentrationen
von Calciumionen und Wahl eines sehr hohen oder
niedrigen pH-Wertes, noch verstärkt werden.
Diese Störungen bewirken, daß sich in der Membrankontaktzone Löcher bilden und es damit zur Ausbildung von Phospholipidbrücken zwischen den
benachbarten Zellmembranen kommt. Dies führt
zur Verschmelzung der beiden Zellen unter Ausbildung einer neuen Einheit.

Bei der Durchführung der bekannten Verfahren
kann jedoch die Zahl der miteinander zu verschmelzenden Zellen nicht gesteuert werden. Einerseits
führt eine zu geringe Zelldichte in der die
Zellen enthaltenden Lösung praktisch zu keinen
Fusionsprodukten, da die Zellen nicht in Kontakt
zueinander kommen. Andererseits führt eine für
eine Fusion hinreichende Zelldichte - die auch
durch Zentrifugieren der die Zellen enthaltenden
Lösung erreicht werden kann - in unkontrollierter
Weise zu Produkten, die aus dem Zwei-, Drei-,
Vier- oder Vielfachen von individuellen Zellen
bestehen.

0128567

Insbesondere im Hinblick auf die Bildung von Hybridzellen durch Verschmelzung von nur zwei Zellen unterschiedlicher Herkunft sind die vorgenannten bekannten Verfahren sehr unzufriedenstellend. Denn Zellhybride, die eine neuartige Kombination von Eigenschaften aufweisen, müssen nach der Durchführung der vorgenannten bekannten Verfahren erst durch die Anwendung sehr zeitraubender Selektionsmethoden isoliert werden. Die Gewinnung von Zellhybriden aus Pflanzenzellen, aus denen wieder ganze Pflanzen gezüchtet werden können oder von Zellhybriden aus tierischen Zellen, über die monoklonale Antikörper, z.B. gegen Tumore und Leukämie, gewonnen werden können, erfordert eine Fusionstechnik, bei der jeweils nur zwei Zellen miteinander fusionieren.

Es ist Aufgabe der Erfindung, ein Verfahren zur Fusion von Zellen zu schaffen, das - unter Einsatz der bekannten Verfahren - eine kontrollierte Fusion zweier oder einer vorbestimmten Anzahl von Zellen ermöglicht.

Diese Aufgabe wird gemäß der Erfindung dadurch gelöst, daß die Zellen zusätzlich zur Behandlung mit den die Störungen bewirkenden Mitteln einem äußeren, inhomogenen elektrischen Feld einer Frequenz von 5 kHz bis 2 MHz und einer Stärke von 10 V/cm bis 2000 V/cm je nach Größe der Zellen derart ausgesetzt werden, daß sich die Zellen in vorbestimmter Ausrichtung aneinanderreihen.

Die Ausrichtung von Zellen im elektrischen Wechselfeld ist aus Biochimica et Biophysica Acta,

- 5 -

694 (1982), 227 - 277 (Elektric Field-Mediated
Fusion And Related Elektrical Phenomena, U. Zimmermann) bekannt. Bei diesem bekannten Verfahren
- dessen Ablauf unter dem Mikroskop beobachtet
werden kann - wird der Membrankontakt zwischen
wenigstens zwei Zellen durch Anlegen eines alternierenden, schwach inhomogenen Feldes erzeugt.
Die Zellen befinden sich dabei in einer Kammer,
bei der ein aus elektrisch nicht leitenden Wänden
gebildeter Raum zur Aufnahme der die Zellen
enthaltenden Suspension vorgesehen ist. In den
Raum der Kammer ragen wenigstens zwei Elektroden
(beispielsweise aus Platindraht) derart hinein,
daß ein zwischen den Elektroden begrenzter Bereich
gebildet wird, in welchem die Zellen dem zwischen
den Elektroden ausgebildeten elektrischen Wechselfeld ausgesetzt sind. Durch das elektrische
Feld werden, bedingt durch Polarisationsprozesse
in der Zelle, Dipole erzeugt, die sich gegenseitig
anziehen, wenn sich die Zellen während ihrer
Wanderung im elektrischen Feld einander nähern
(sog. Dielektrophorese).

Durch die Verfahrensweise gemäß der Erfindung
wird nicht nur eine kontrollierte Fusion zweier
oder einer vorbestimmten Anzahl von Zellen bei
Einsatz chemischer Mittel oder inaktivierter
Viren ermöglicht. Vielmehr ist darüberhinaus
bei Anwendung des Verfahrens gemäß der Erfindung
die Möglichkeit gegeben, die bisher bekannten
Verfahren im Hinblick auf die Dosierung der
die Störungen bewirkenden Mitteln genauer zu

- 6 -

- 6 -

untersuchen bzw. zu optimieren.

Zweckmäßig ist es, daß die Zellen zu ihrer Ausrichtung etwa 2 bis 5 Minuten dem elektrischen Wechselfeld ausgesetzt werden. Diese Zeitspanne genügt im allgemeinen, um die Zellenreihe bzw. die Zellenreihen zu bilden.

Um eine unerwünschte Erwärmung der die Zellen enthaltenden Lösung und damit der Zellen zu vermeiden, wird das elektrische Feld zweckmäßigerweise nach Bildung der Zellenreihe abgeschaltet. Es genügt dann, bis zum Eintritt der Funktion der Zellen die Zellenreihe dadurch aufrechtzuhalten, daß das elektrische Wechselfeld von Zeit zu Zeit für einige Sekunden wieder eingeschaltet wird.

Eine besonders vorteilhafte Ausführungsvariante des Verfahrens gemäß der Erfindung besteht für den Fall, daß als die Störungen bewirkendes Mittel Polyethylenglycol oder Sendai-Viren eingesetzt werden, darin, daß zunächst das Mittel der die Zellen als Suspension enthaltenden Lösung in vorgesehener Dosierung zugegeben wird, danach die Zellenreihe mittels des elektrischen Feldes gebildet wird und sodann Calcium-Ionen zur Bewirkung der Störungen in der Membran der Zellen zugegeben werden. Bei Verwendung von Sendai-Viren wird außerdem die Temperatur auf 37 °C erhöht. Bei dieser Verfahrensweise setzt die Wirkung der Mittel erst nach Ausrichtung der Zellen ein.

- 7 -

- 7 -

Da bei Anwendung des Verfahrens gemäß der Erfindung die Fusion von Zellen in der gebildeten Zellenreihe leicht im Mikroskop beobachtet werden kann, ist es auf einfache Weise möglich, verschiedene Dosierungen der die Störungen bewirkenden Mitteln zu testen und für eine bestimmte Zellenart zu optimalisieren.

Ausführungsbeispiel 1

Erythroleukämische Zellen (sog. Friend-Zellen) wurden in eine isotone, 0.3 molare Mannitlösung inkubiert, der 41,5 % Polyethylenglycol (BEG; MW 6000) sowie 15 % Dimethylsulfoxid (DMSO) zugegeben wurden.

Die Suspension wurde in eine Fusionskammer gegeben, deren Elektrodenabstand 300 µm betrug. Darauf wurde ein Wechselfeld mit einer Feldstärke von 300 V/cm und einer Frequenz von 2 MHz für etwa 3 Minuten bis zur Bildung der Zellenreihe angelegt. Bei der gegebenen Suspensionsdichte der Zellen (etwa $10^3$/ml) bildeten sich dabei bevorzugt Ketten aus zwei oder drei Zellen.

Nach Bildung der Zellenreihe wurde das elektrische Wechselfeld abgeschaltet und der in der Kammer befindlichen, die Zellen enthaltenden Lösung vorsichtig eine Lösung zugegeben, die 10 mM $CaCl_2$ in 0.3 molarer Mannitlösung enthielt. Durch die Zugabe der Calciumionen wurde die Fusion der Zellen innerhalb der Ketten durch PEG ausgelöst.

- 8 -

## Ausführungsbeispiel 2

Wie in Ausführungsbeispiel 1 beschrieben, wurden erythroleukämische Zellen in der Fusionskammer mittels des elektrischen Wechselfeldes zu Zellenreihen ausgerichtet. In der die Zellen enthaltende Lösung war jedoch kein PEG, sondern inaktivierte Sendai-Viren einer Konzentration von etwa 2 x $10^3$ HAU/ml (haemagglutinierende Einheiten) enthalten. Nach Bildung der Zellenreihe wurde das elektrische Wechselfeld abgeschaltet und die doppelte Menge einer Lösung zugegeben, die etwa 4 mM/l $CaCl_2$ in phosphatgepufferter isotoner NaCl-Lösung enthielt. Darauf folgende Temperaturerhöhung auf 37 °C löste Fusion aus.

Kernforschungsanlage Jülich
Gesellschaft mit beschränkter Haftung

P a t e n t a n s p r ü c h e

1. Verfahren zur Fusion von Zellen, bei dem die Zellen zur Erzeugung von Störungen in der Membranstruktur, die zur Bildung von Membranbrücken zwischen einander benachbarten Zellen und zur Fusion der Zellen führen, mit chemischen Mitteln, wie Polyethylenglycol und Calciumionen, oder inaktivierten Viren, wie Sendai-Viren, behandelt werden, d a d u r c h   g e k e n n z e i c h - n e t , daß die Zellen zusätzlich zur Behandlung mit den die Störungen bewirkenden Mitteln einem äußeren, inhomogenen elektrischen Feld einer Frequenz von 5 kHz bis 2 MHz und einer Stärke von 10 V/cm bis 2000 V/cm je nach Größe der Zellen derart ausgesetzt werden, daß sich die Zellen in vorbestimmter Ausrichtung aneinanderreihen.

2. Verfahren nach Anspruch 1, d a d u r c h   g e k e n n z e i c h n e t , daß die Zellen zur Bildung der Zellenreihe etwa 2 bis 5 Minuten dem elektrischen Wechselfeld ausgesetzt werden.

3. Verfahren nach einem der Ansprüche 1 oder 2, d a d u r c h   g e k e n n z e i c h n e t , daß nach Bildung der Zellenreihe das elektrische Wechselfeld abgeschaltet und lediglich zur Aufrechterhaltung der Zellenreihe für jeweils einige Sekunden wieder eingeschaltet wird.

- 2 -

- 2 -

4. Verfahren nach einem der vorhergehenden Ansprüche, d a d u r c h  g e k e n n z e i c h n e t , daß als die Störungen bewirkendes Mittel Polyethylenglycol der die Zellen enthaltenden Lösung zugegeben wird, danach die Zellenreihe mittels des elektrischen Wechselfeldes gebildet wird und sodann Calciumionen zur Bewirkung der Störungen in der Membran der Zellen zugegeben werden.

5. Verfahren nach einem der Ansprüche 1 bis 3, d a d u r c h  g e k e n n z e i c h n e t , daß als die Störungen bewirkendes Mittel inaktivierte Sendai-Viren zugegeben werden, danach die Zellenreihe mittels des elektrischen Wechselfeldes gebildet wird und sodann Calciumionen zur Bewirkung der Störungen in der Membran der Zellen zugegeben werden.